Europäisches Patentamt

European Patent Office

Office européen des brevets

(11) Publication number : **0 508 894 A1**

## (12) EUROPEAN PATENT APPLICATION

(21) Application number : **92400990.5**

(22) Date of filing : **09.04.92**

(51) Int. Cl.⁵ : **E21B 47/10, G01N 33/28**

(30) Priority : **11.04.91 FR 9104406**

(43) Date of publication of application :
**14.10.92 Bulletin 92/42**

(84) Designated Contracting States :
**DE GB NL**

(71) Applicant : **SCHLUMBERGER LIMITED**
**277 Park Avenue**
**New York, N.Y. 10172 (US)**
(84) **GB**

(71) Applicant : **SCHLUMBERGER TECHNOLOGY**
**B.V.**
**Carnegielaan 12**
**NL-2517 KM Den Haag (NL)**
(84) **DE**

(71) Applicant : **SCHLUMBERGER HOLDINGS**
**LIMITED**
**P.O. Box 71, Craigmuir Chambers**
**Road Town, Tortola (VG)**
(84) **NL**

(72) Inventor : **Vigneaux, Pierre**
**36, rue Grande**
**F-77950 Moisenay (FR)**

(74) Representative : **Hagel, Francis et al**
**Etudes et Productions Schlumberger A**
**L'ATTENTION DU SERVICE BREVETS 26, rue**
**de la Cavée B.P. 202**
**F-92142 Clamart Cédex (FR)**

(54) **A method of locally determining the nature of a phase in a three-phase fluid, and application for determining flow parameters of the fluid.**

(57)    In order to determine the flow parameters of a three-phase fluid which e.g. in a hydrocarbon producing well, may be constituted for example by a continuous water phase together with discontinuous phases of oil and of gas in the form of bubbles, a local sonde (40a) is used for identifying the phases present. The sonde (40a) comprises two sensors (42a, 44) which are responsive to different physical properties of the fluid and which are situated at substantially the same point, in such a manner as to provide a different set of signals for each of the phases. The sensors may comprise, in particular a radiofrequency sensor and an optical sensor disposed coaxially therewith at the end of a coaxial cable (42a) whose core comprises a metal-coated optical fiber (44).

FIG. 2A

EP 0 508 894 A1

The invention relates to a method suitable for use with a flowing fluid that may contain three distinct phases, namely a continuous phase and dispersed phases, to determine the nature of the phase at a specific location, e.g. in a hydrocarbon producing well.

The invention also relates to a method and to apparatus capable, after the phase in presence has been identified, of determining the flow parameters of the fluid such as the velocity and the diameter of the bubbles present in the continuous phase, or the superficial velocity of the dispersed phase.

In a hydrocarbon well in production, the fluid recovered at the surface may comprise a mixture of water - which generally forms the continuous phase - and of oil and gas which form the dispersed phases. For well monitoring, it is desirable to know the respective proportions of each of the phases at various different depths. To this effect, it is common practice to perform production logging measurements.

In the case of a two-phase fluid, French Patent 2 645 901 proposes placing one or more very small local sensors, such as radiofrequency sensors, at successive different depths inside a hydrocarbon well. In that case, the output signal from each sensor is at a different level depending on whether the sensor is in the presence of water or of hydrocarbon. In addition, it has been observed that the transition periods between the various levels of each signal can be used to determine some of the flow parameters of the dispersed phase which is usually in the form of bubbles in the continuous phase. These transition periods can be used, in particular, to determine the displacement velocities of the bubbles, and in combination with a signal representative of the length of time a bubble is present at a sensor, to determine the diameters of the bubbles and also the superficial velocity of the dispersed phase, i.e. the ratio of the flow rate of said phase to the measurement section.

In practice, these various items of information are obtained after the measurement signal provided by the local sensor has been processed. The processing provides a first signal indicative of the length of time a bubble is present at the sensor, and a second signal indicative of the durations of the transition periods during which the output signal from the sensor changes from one level to another.

When the fluid flowing along the hydrocarbon well contains only one dispersed phase such as oil within a continuous phase such as water, the use of small-sized sensors (radiofrequency sensors for a water-oil mixture) makes it possible for the measurements mentioned above to be performed under good conditions.

However, such sensors do not enable the expected results to be achieved when the flowing fluid contains a second dispersed phase such as a gas which a radiofrequency sensor is incapable of distinguishing from oil in the presence of water.

According to the invention, a local determination of the nature of a phase in a moving three-phase fluid is achieved by using information provided by a sonde constituted by two sensors that are different in nature, the sensors being small in size and located at substantially the same position.

More precisely, the present invention provides a method of locally determining the nature of a phase in a flowing fluid that may contain three distinct phases, comprising the steps of :

generating at least two signals at substantially the same point in the fluid, said signals being respectively representative of the local values of different physical properties of the fluid at said point, said properties being such that each phase corresponds to a distinctive set of signals;

detecting said signals; and

identifying the nature of the phase from the set of detected signals.

Advantageously, a first one of the signals is obtained by means of a radiofrequency sensor capable of measuring the local dielectric constant of the fluid, and making it possible to distinguish the presence of hydrocarbon (oil or gas) in the water.

The second signal may be provided by any other sensor capable of distinguishing a gaseous phase from a liquid phase (water or oil). In particular, such a sensor may be an optical sensor responsive to the refractive index of the fluid, or a sensor responsive to pressure fluctuations in the fluid, such as a piezoelectric sensor responsive to the propagation velocity of sound in the fluid.

Advantageously, these two sensors may be mounted coaxially so as to form a single sonde capable of performing two different measurements at substantially the same point in the fluid.

Thus, in a first embodiment of the invention, the sonde comprises a coaxial cable having one end forming a radiofrequency sensor, said cable including a metal-coated central optical fiber having one end that constitutes both an optical sensor and a central conductor for the radiofrequency sensor.

In a second embodiment of the invention, the sonde comprises a coaxial cable having one end constituting a radiofrequency sensor, the said cable including an intermediate tube made of electrically insulating material with one end thereof constituting both an optical sensor and an insulator of the radiofrequency sensor.

Once the nature of a phase has been established, the invention also makes it possible to determine the flow parameters of a fluid that may contain three distinct phases, on the basis of the measurement signal delivered by at least one of the sensors of the sonde.

To this end, once the nature of the phase present has been identified, at least one of the measurement signals delivered by the two sensors is processed for the purpose of obtaining at least one signal representative of a flow parameter of said phase.

A preferred embodiment of the invention is described by way of non-limiting example and with reference to the accompanying drawings, in which:

Figure 1 is a diagrammatic side view, partially in section, showing a logging installation installed at an oil well for performing the methods of the invention and constituting the apparatus of the invention;

Figures 2A and 2B are longitudinal section views showing two different embodiments of a sonde designed for use in the installation of Figure 1, in accordance with the invention;

Figure 3 is a block diagram showing the various circuits which are associated with the sonde to identify the phase that is present and to determine the various physical parameters relating to the flow of the fluid; and

Figure 4 is a waveform diagram showing one example of how the measurement signals $V_{RF}$ and $V_{OP}$ delivered by the detector circuits associated with each of the sensors in the sonde vary simultaneously at a function of time t as a bubble of oil and a bubble of gas pass the sonde in succession.

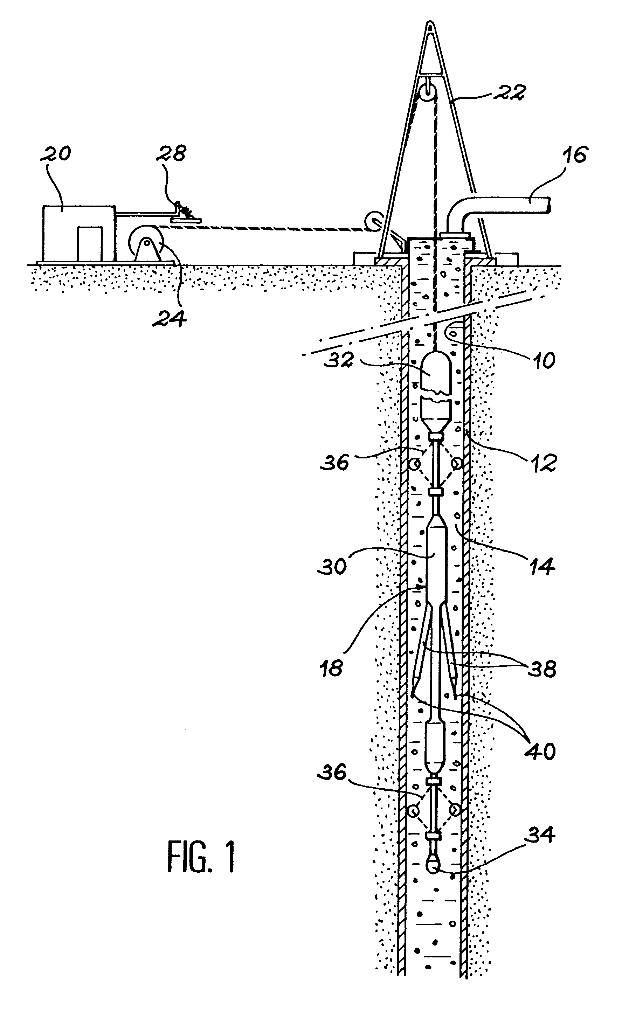

Figure 1 shows an oil well 10 lined internally with cylindrical casing 12 through which a three-phase fluid 14 flows towards the surface. In the description below, and by way of example, the three-phase fluid is assumed to be constituted by a continuous water phase having a dispersed phase of oil and a dispersed phase of gas therein, both in the form of bubbles. The fluid reaching the surface is conveyed by a duct 16 to a storage installation (not shown).

The well 10 shown in Figure 1 is also fitted with a logging installation mainly comprising a logging tool 18 suitable for being displaced within the casing 12 to perform measurements at different depths, and a surface unit 20 comprising means for exploiting the information delivered by the logging tool 18.

The logging tool 18 is suspended from the end of a cable 22 whose opposite end is wound onto a winch 24. To obtain the depth of the logging tool 18 down the casing 12, detector means 28 known to the person skilled in the art are associated with the winch 24 for detecting magnetic marks that are disposed at regular intervals along the cable 22.

The logging tool 18 mainly comprises an elongate body 30 whose top and bottom ends are respectively connected to an electronics section 32 and to a nose 34. Positioning members 36 such as centralizers are placed immediately above and below the body 30 so as to position it inside the casing 12 when a measurement is to be performed. In the example shown, the members 36 serve to place the elongate body 30 on the axis of the casing. Alternatively, other types of positioning member could be used, in particular for pressing the elongate body 30 against the casing.

The body 30 of the logging tool 18 carries one or more hinged arms 38 (e.g. three arms) pivotally mounted about axes that are orthogonal to the longitudinal axis of the tool, whereby each of them is movable between a position in which it is retracted against the body 30 and a deployed position as shown in Figure 1. The retracted position of the arms 38 is used while the tool is being displaced between two measurements stations. In contrast, the arms 38 which carry measurement sondes 40 at their ends are placed in their deployed positions when a measurement is to be performed. The pivoting of the arms 38 between their two positions may be controlled by any conventional means such as hydraulic actuators (not shown).

The positions occupied by the local sondes 40 in the well depend both on the position of the body 30 inside the casing 12 as determined by the positioning members 36, and on the deployed positions of the arms 38, with said positions being advantageously determined as described and claimed in French Patent 2 637 089.

In accordance with the invention, each of the local measurement sondes 40 comprises two sensors that are mounted coaxially so as to measure two different physical characteristics of the fluid 14 at a single location. More precisely, these physical characteristics are selected so that the two sensors are capable of performing different discriminations between the three phases contained in the fluid. In particular, in the example under consideration where the fluid 14 comprises a continuous water phase containing bubbles of oil and bubbles of gas, one of the sensors is selected to be capable of distinguishing between hydrocarbons (oil or gas) and water, while the other sensor is selected to be capable of distinguishing between gas and liquid (water or oil).

In practice, the first sensor may be constituted, in particular, by a radiofrequency sensor responsive to changes in the dielectric constant of the medium in which it is located. The second sensor may be constituted by an optical sensor which detects changes in the refractive index of the medium.

In a variant, the second sensor may alternatively be an anisotropic optical fiber sensor including a component whose plane of polarization varies as a function of pressure. The second sensor could also be implemented in the form of a stack of piezoelectric pellets made of ceramic and responsive to the propagation of sound in the medium.

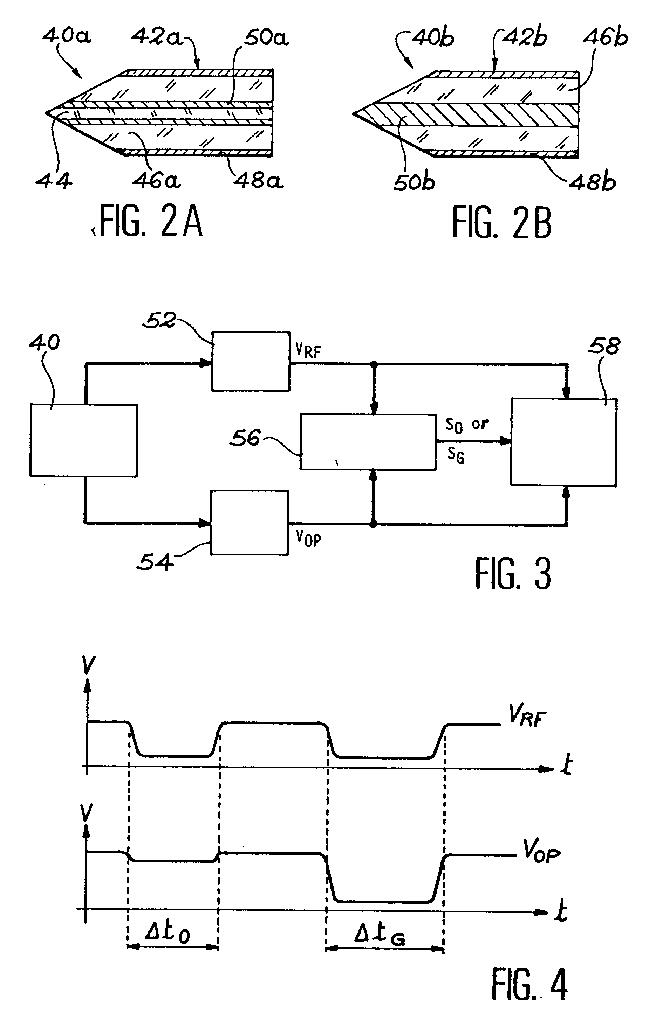

Two embodiments of a sonde comprising an association of a radiofrequency sensor and an optical sensor responsive to the refractive index of the medium are described below with reference to Figures 2A and 2B in succession.

In the embodiment shown in Figure 2A, the sonde 40a is formed by a conical end of a coaxial cable 42a whose central core is a metal-coated optical fiber 44. The conical end of the coaxial cable 42a constitutes a radiofrequency sensor, whereas the conical end of the optical fiber 44 forms an optical sensor.

More precisely, the coaxial cable 42a comprises: an electrically insulating intermediate component, e.g. constituted by a glass tube 46a; an outer conductor 48a, e.g. constituted by a layer of molybdenum; and a central conductor 50a constituted by a metal coating, e.g. of molybdenum, formed on the optical fiber 44. The optical fiber 44 may itself be constituted by a silica fiber. In order to ensure that the assembly withstands pressure and above all to avoid any risk of fluid infiltrating into the interfaces between the various layers of the sonde 40a, in particular between the metal coating 50a and the insulating component 46a, the parts of the assembly may be interconnected by gluing or by melting a metal coating 50a.

A combined optical and electronic connector (not shown) of known structure connects the opposite end of the coaxial cable 42a to the detector circuit associated with the radiofrequency sensor, which circuit is represented diagrammatically at 52 in Figure 3. In conventional manner and as described in detail in French Patent 2 645 901, this detector circuit comprises a high frequency generator whose output is connected to an amplifier associated with a lowpass filter, with this assembly being connected to the end of a branch of a Wheatstone bridge having one of its branches constituted by the coaxial cable 42. A detector located at the center of the bridge delivers a measurement signal $V_{RF}$ representative of bridge unbalance. This unbalance is itself a function of the dielectric constant of the phase of the fluid in contact with the end of the sonde 40a. Thus, and as shown at the top of Figure 4, the measurement signal $V_{RF}$ is significantly lower during time intervals $\Delta t_O$ and $\Delta t_G$ during which the end of the sonde is respectively in contact with a bubble of oil and with a bubble of gas, than when said end is in contact with the continuous water phase.

As mentioned above, the optical sensor is constituted by the conically pointed end of the optical fiber 44. The cone angle formed by said end depends on the dielectric constants of the various phases of the fluid in which the sensor is located. Because of this angle, a highly directional incident light beam conveyed by the optical fiber 44 is totally reflected with a phase or phases having a refractive index greater than a given threshold whereas, in contrast, it is totally transmitted with a phase or phases having a refractive index below the threshold.

In practice, the optical sensor allows bubbles of gas to be distinguished from the liquid phases (water and oil) present in the fluid flowing along the well. Suitably, the angle formed by the end cone of the optical fiber 44 relative to a right cross-section through the fiber is given a value that is close to 65°.

As shown diagrammatically in Figure 3, the optical sensor made of the conical end of the optical fiber 44 is connected by the fiber to an associated detector circuit 54. The detector circuit 54 essentially comprises a laser diode that emits a beam of substantially parallel light which is transmitted to the optical sensor by the optical fiber, and a photoreceiver capable of transforming the light beam that may be reflected by the conical end of the fiber into an optical measurement signal $V_{OP}$.

The bottom portion of Figure 4 shows that this measurement $V_{OP}$ has a substantially constant value while the sensor is in the continuous water phase and while it is in the presence of a bubble of oil during time interval $\Delta t_O$. This value is a high value corresponding to substantially total reflection of the parallel light beam at the conical end of the optical fiber and representative of a liquid medium whose refractive index is greater than a threshold determined by the angle formed at said conical end.

In contrast, during the presence of a bubble of gas during time interval $\Delta t_G$, the measurement signal $V_{OP}$ delivered by the detection circuit 54 associated with the optical sensor drops to take up a value that is substantially zero, which represents substantially total transmission of the light beam at the conical end of the optical fiber 44. In this case, the refractive index of the gas is below the threshold beyond which the incident light beam is totally reflected by the conical end of the optical fiber 44.

In the sonde structure described above with reference to Figure 2A, it is important to observe that proper operation of the radiofrequency sensor merely requires the antenna-forming central conductor 50a to project beyond the end of the outer conductor 48a. For reasons concerned with ease of manufacture and with obtaining good mechanical strength, and given the conical shape of the end of the optical fiber 44, it is advantageous for the entire end of the sonde to be given a conical shape as shown in Figure 2A.

However, this configuration could possibly be modified, e.g. by extending the insulating component 46a to the end of the central conductor 50a and cutting it in accordance with a cross-section at that point.

Figure 2B shows a second embodiment of a sonde capable of being used in the installation described above with reference to Figure 1. In order to distinguish it from the sonde 40a described above with reference to Figure 2A, this sonde is given reference 40b.

As in the first embodiment, the sonde 40b comprises a radiofrequency sensor and an optical sensor which are disposed coaxially.

The radiofrequency sensor is constituted by the end of a coaxial cable 42b which comprises an antenna-forming central conductor 50b, an outer conductor 48b, and a tubular insulating component 46b disposed between the conductors 48b and 50b. In this case, the antenna-forming central conductor 50b is a solid conductor, e.g. made of molybdenum. As in the first embodiment, the outer conductor 48b may also be made of molybdenum. The insulating component 46b is made of glass or of silica and, in this case, it constitutes an annular optical fiber whose end constitutes the optical sensor of the sonde 40b.

As in the first embodiment, the end of the coaxial cable 42b is advantageously conically pointed so as to ensure firstly that the antenna-forming central conductor 50b projects beyond the end of the outer conductor 48b, and secondly that the end of the insulating component 46b constituting the optical sensor is conically pointed at an angle of about 65° relative to a cross-section through the coaxial cable.

As in the first embodiment, the opposite end of the coaxial cable 42 is connected via a combined optical and electronic connector firstly to the detector circuit associated with the radiofrequency sensor and secondly to the detector circuit associated with the optical sensor, as shown highly diagrammatically in Figure 3.

The detector circuit associated with the radiofrequency sensor is identical to that described above for the sonde 40a shown in Figure 2A.

In contrast, given the annular nature of the optical fiber connecting the optical sensor to the associated detector circuit, the laser diode used in the first embodiment is replaced in this case by a lamp constituting a light source that emits a beam of light that is not parallel. Multiple reflections then occur during transmission of this incident beam along the optical fiber. As before, the incident light beam is reflected at the end of the optical fiber when the refractive index of the phase in which said end is immersed is greater than a given threshold. In contrast, the beam is transmitted through said end towards the fluid medium when the refractive index of the phase in which the end of the fiber is immersed is less than the threshold.

However, given the non-directional nature of the light beam conveyed by the optical fiber, reflection in the first case is not total, and in the second case transmission is not total. Consequently, the signal $V_{OP}$ delivered in this case by the photosensor of the detector circuit associated with the optical sensor presents, as comared with the first embodiment of the sonde, a smaller difference in level between portions corresponding to the sonde being present in a liquid phase (water or oil) of refractive index greater than the predetermined threshold, and in a gaseous phase of refractive index below the threshold. This reduced sensitivity is nevertheless acceptable provided the associated electronics is capable of distinguishing without ambiguity between the associated corresponding portions of the measurement signal from the optical sensor.

The non-directional nature of the light beam conveyed by the optical fiber constituted by the insulating component 46b also has the consequence that the operation of the optical sensor is less sensitive to the angle formed at the end of the optical fiber relative to a right cross-section of the coaxial cable 42b. This may make it possible to give to the end of the optical fiber constituted by the dielectric 46b a shape that is slightly different from a cone, e.g. a shape that is rounded or spherical.

The embodiment shown in Figure 2B also has the advantage over the preceding embodiment of being simpler in structure, thereby facilitating manufacture and making it easier to obtain good pressure resistance properties, and above all good sealing.

It should be observed that in each of the embodiments described with reference to Figures 2A and 2B, the mechanical strength of the end of a coaxial cable constituting the sonde per se can be improved by replacing the insulating component 46a at this point by a part made of a material that is stronger but that has the same electrical and optical characteristics, e.g. sapphire. However, in the case of the second embodiment shown in Figure 2B, this solution creates an additional interface on the path of the incident light beam and on the path of the reflected light beam.

As shown diagrammatically in Figure 3, the measurement signals $V_{RF}$ and $V_{OP}$ delivered by the detector circuits 52 and 54 respectively associated with the radiofrequency sensor and with the optical sensor are applied to a circuit 56 for identifying which phase is present. The circuit 56 firstly processes each of the measurement signals $V_{RF}$ and $V_{OP}$ to obtain respective binary signals having the value zero when the corresponding measurement signal is greater than a determined threshold and the value one when the same signal is below said threshold. Thus, from the measurement signal $V_{RF}$, a signal is obtained having rectangular pulses for periods of time such as $\Delta t_O$ and $\Delta t_G$ respectively representative of a bubble of oil being present and a bubble of gas being present at the sonde, and from the measurement signal $V_{OP}$, a signal is obtained having rectangular pulses during each period of time such as $\Delta t_G$ during which the sonde "sees" a bubble of gas.

On the basis of these rectangular type signals, the circuit 56 for identifying which phase is present can identify the phase in which the sonde is located by means of a logic circuit that is particularly simple. When neither of the logic signals derived from the measurement signals $V_{RF}$ and $V_{OP}$ has a pulse, then the sonde is in the continuous water phase, when a pulse is present on both of the logic signals, then the sonde is present in a bubble of gas, and when a pulse is present only in the logic signal derived from the measurement signal $V_{RF}$, then the sonde is in a bubble of oil.

The circuit 56 also indicates which of the phases present forms the continuous phase (except when the fluid contains water and gas only, in which case the responses of the two sensors are the same).

The information obtained in this way by the circuit 56 concerning the fluid phase in which the sonde is located is transmitted to a processor circuit 58, e.g. in the form of a signal $S_O$ when the sonde is in a bubble of oil and in the form of a signal $S_G$ when the sonde is in a bubble of gas.

The processor circuit 58 which, it should be observed, is formed by a single circuit for both of the sensors of the sonde 40, also receives each of the measurement signals $V_{RF}$ and $V_{OP}$. By applying a pre-established program (which may depend, in particular, on which of the two signals $S_O$ and $S_G$ is received), the processor circuit 58 can make use of either one only of the two measurement signals $V_{RF}$ and $V_{OP}$, or else it may make use of both of these signals simultaneously.

On the basis of at least one of the measurement signals $V_{RF}$ and $V_{OP}$, the processor circuit 58 serves to determine various parameters concerning the flow of fluid present in the well, at the level at which the logging tool 18 of Figure 1 is located. To do this, it is advantageous to process that one of the measurement signals which has the maximum amplitude variation for the phase that is present. The flow parameters may be detected, in particular, in the manner described in french Patent 2 645 901.

As described in that document, it is possible to deduce from at least one of the measurement signals a signal that is representative of the time interval required for the measurement signal in question to switch from one level to the other. The gradient of the measurement signal is then calculated, which gradient is proportional to the displacement velocity of the bubble in question.

The processor circuit 58 also derives from the measurement signal in question a signal that is representative of the time that a bubble is present at the sensor. This signal is then combined with the signal representative of the gradient of the measurement signal to obtain a signal which is representative of the diameter of the bubble in question.

Finally, the processor circuit 58 serves to totalize the signals representative of bubble diameter over a unit of time to obtain the superficial velocity of the dispersed phase under consideration (oil or gas), which corresponds to the local flow rate of this phase assumed to be on its own spread over the section of the well at that level.

It should be observed that the various different forms of processing that may be performed by the circuit 58 are given purely by way of example, and other processing may be envisaged without going beyond the scope of the invention.

It may also be observed that although the invention is particularly suitable for performing measurements in an oil well, other applications are possible, particularly in the chemical industry, so long as there is a fluid containing at least three different phases flowing along pipework and it is desired to establish certain characteristics of the flow of said fluids. From this point of view, it should also be observed that the phases constituting the fluid may be different from the three phases described by way of example, providing the two small-sized sensors located at substantially the same location are capable of distinguishing the three phases by measuring different physical characteristics of the fluid.

## Claims

1. A method of locally determining the nature of a phase in a flowing fluid that may contain three distinct phases, comprising the steps of:

   generating at least two signals at substantially the same point in the fluid, said signals being respectively representative of the local values of different physical properties of the fluid at said point, said properties being such that each phase corresponds to a distinctive set of signals;

   detecting said signals; and

   identifying the nature of the phase from the set of detected signals.

2. A method according to claim 1, wherein a first one of the signals is representative of the dielectric constant of the phase.

3. A method according to claim 1 or 2, wherein a second one of the signals is representative of a physical property selected from the group comprising: the refractive index of the fluid; the pressure of the fluid; and the propagation velocity of sound in the fluid.

4. A method according to claim 3, wherein said signals are generated by placing a sonde in the fluid, the sonde comprising a radiofrequency sensor and an optical sensor that are mounted coaxially.

5. A method of determining the flow parameters of a fluid that may contain three distinct phases, comprising the steps of:

   generating at least two signals at substantially the same point in the fluid, said signals being respectively representative of the local values of different physical properties of the fluid at said point, said properties being such that each phase corresponds to a distinctive set of signals;

   detecting said signals;

   identifying the nature of the phase from the set of detected signals; and

   processing at least one of said measurement signals to obtain at least one processed signal representative of a flow parameter of the phase that is present.

6. A method according to claim 5, wherein the measurement signal which is processed is that one of the measurement signals which has the largest amplitude variation for said phase that is present.

7. A method according to claim 5 or 6, wherein the measurement signals are generated by placing a sonde in the fluid, the sonde comprising a radiofrequency sensor and an optical sensor disposed coaxially.

8. Apparatus for determining the flow parameters of a fluid that may contain three distinct phases, comprising a sonde including two local sensors located at the same point and responsive to respective different physical properties of the fluid, to deliver signals that have different levels depending on the phase which is in contact with the sonde, said signals constituting a different set for each of the phases of the fluid.

9. Apparatus according to claim 8, wherein the sonde comprises a radiofrequency sensor and an optical sensor mounted coaxially.

10. Apparatus according to claim 9, wherein the sonde comprises a coaxial cable having one end constituting the radiofrequency sensor, the coaxial cable including a metal-coated central optical fiber having one end which constitutes both the optical sensor and a central conductor of the radiofrequency sensor.

11. Apparatus according to claim 9, wherein the sonde comprises a coaxial cable having one end that forms the radiofrequency sensor, said coaxial cable including an intermediate tube made of an electrically insulating material, and having one end which constitutes both the optical sensor and an insulator of the radiofrequency sensor.

FIG. 1

EP 0 508 894 A1

FIG. 2A

FIG. 2B

FIG. 3

FIG. 4

9

**European Patent Office**

**EUROPEAN SEARCH REPORT**

Application Number

EP    92 40 0990

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | DE-B-1 213 814 (SCHLUMBERGER)<br>* claim 1 *<br>--- | 1-3 | E21B47/10<br>G01N33/28 |
| X<br>Y | DD-A-221 635 (R.JOHANNS ET AL.)<br><br>* the whole document *<br>--- | 8<br>1-3 | |
| D,X | FR-A-2 645 901 (SCHLUMBERGER)<br>* page 5, line 33 - page 10, line 10 *<br>--- | 5,6 | |
| P,A | EP-A-0 435 623 (CASTROL LTD.)<br>* the whole document *<br><br>----- | 1,2,5 | |
| | | | **TECHNICAL FIELDS SEARCHED (Int. Cl.5 )**<br><br>E21B<br>G01N |

The present search report has been drawn up for all claims

| Place of search | Date of completion of the search | Examiner |
|---|---|---|
| THE HAGUE | 18 JUNE 1992 | RAMPELMANN K. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...................................................................
& : member of the same patent family, corresponding
   document

EPO FORM 1503 03.82 (P0401)